# EUROPEAN PATENT SPECIFICATION

(11) **EP 1 656 099 B1**
(45) Date of publication and mention of the grant of the patent: **09.04.2008**
(21) Application number: 04757414.0
(22) Date of filing: 02.08.2004
(51) Int. Cl.: A61K 8/00, A61Q 5/00, A61Q 19/00

(54) **EMULSIONS WITH A CONCENTRATED INTERNAL OIL PHASE**
EMULSIONEN MIT EINER KONZENTRIERTEN INNEREN ÖLPHASE
EMULSIONS PRESENTANT UNE PHASE HUILEUSE INTERIEURE CONCENTREE

(30) Priority: 07.08.2003 US 493279 P
(43) Date of publication of application: 17.05.2006
(73) Proprietor: THE PROCTER & GAMBLE COMPANY, Cincinnati, Ohio 45202 (US)
(72) Inventor: DECKNER, George, Endel, Cincinnati, OH 45249 (US)
(74) Representative: Wilding, Richard Alan
(86) International application number: PCT/US2004/024896
(87) International publication number: WO 2005/016294

(56) References cited:
- EP-A- 1 319 388
- US-A1- 2002 035 182
- US-A1- 2002 090 346
- SOAP AND COSMETICS, vol. 77, no. 9, 2001, pages 39-43, XP001203606
- SOAP AND COSMETICS, vol. 77, no. 7, 2001, pages 43-46, XP001203607
- DALLAL J: "POLYQUATERNIUM-55: A NEW FORCE IN STYLING POLYMERS" COSMETICS & TOILETRIES, WHEATON, IL, US, vol. 117, no. 12, December 2002 (2002-12), pages 33-34,36, XP009024595 ISSN: 0361-4387

## Description

### FIELD OF INVENTION

The present application concerns oil-in-water emulsions having a concentrated internal phase and products, such as cosmetic products, comprising the emulsions.

### BACKGROUND OF INVENTION

Emulsions are generally stabilised by appropriate emulsifying surfactants, which, by virtue of their amphiphillic structure, reside at the oil/water interface and thus stabilise the dispersed droplets. These surfactants typically exhibit the disadvantage, however, of penetrating and potentially irritating the skin, eyes and scalp and generally give poor skin feel. Furthermore, the use of conventional surfactants to manufacture emulsions typically necessitates the application of heat during processing, which can also be disadvantageous, in that it can restrict the ability to include heat-sensitive ingredients and in that it may also limit the types of place in which the manufacturing method may be performed - safety and other concerns, may, for example, prohibit manufacturing the emulsions in certain desired locations.

Another disadvantage of traditional surfactants, including alkoxylated surfactants, is that they may cause materials to re-emulsify after the emulsion breaks - emulsion breakage allows delivery of emulsified materials, but re-emulsification, such as after application of a personal cleansing composition to the skin during washing/showering, may reduce the desired benefit (because emulsified emollients and actives are washed off the skin in this example).

A further disadvantage of conventional surfactants is their inability to satisfactorily emulsify polar oils, such as oils having a high solubility parameter.

Concentrated emulsions having a high discontinuous phase, wherein the discontinuous phase comprises water or oil, for example, are known and have found application in a number of technologies, such as fuels, cosmetics and foods - an everyday example of these emulsions is mayonnaise (which may typically comprise about 70% vegetable oil in water). These concentrated emulsions have also found application in the cosmetic area because the concentrates can stably contain high concentrations of, for example, emollients, moisturisers and sunscreens, which can then be diluted down using simple cold mixing to obtain the desired end product. Reference may be made to US 4,606,913 and US 5,976,604, which teach concentrated emulsions.

In the light of the above considerations, it would be beneficial to develop oil-in-water emulsions which have a reduced capacity to irritate human skin and membranes and provide improved skin feel. In addition, it would be advantageous to develop emulsions which are more substantive to the substrate to which they are applied, such as human skin or fabrics, and exhibit a reduced tendency to re-emulsify once broken.

### SUMMARY OF INVENTION

According to a first aspect of the invention, an emulsion is provided comprising at least 50% by weight of the emulsion of discontinuous oil phase, an aqueous continuous phase and emulsifier, wherein the emulsifier comprises a non-alkoxylated water-soluble emulsification polymer having a molecular weight of at least 500 Daltons, a 0.1%wt aqueous solution of the water-soluble emulsification polymer having a surface tension of 15-60 mN/m (15-60 dynes/cm) when measured at 25°C.

As used herein, the term "non-alkoxylated" in relation to the water-soluble emulsification polymers means polymers comprising no alkoxy groups, that is no -OR groups (where R includes alkyl moieties) in the molecule, neither in the polymer backbone, nor as pendants thereto nor elsewhere.

As used herein, the term "oil-in-water" or "o/w" means that an oil phase is dispersed in an aqueous phase, such that the aqueous phase is the continuous phase and the oil phase the discontinuous phase.

According to a second aspect of the invention, personal care, health care and laundry products are provided comprising from 0.01 to 30%wt, preferably from 0.25 to 5%wt of the emulsion according to the first aspect of the invention.

### DETAILED DESCRIPTION OF THE INVENTION

All weights, measurements and concentrations herein are measured at 25°C on the composition in its entirety, unless otherwise specified.

Unless otherwise indicated, all percentages of compositions referred to herein are weight percentages of the total composition (i.e. the sum of all components present) and all ratios are weight ratios.

Unless otherwise indicated, all polymer molecular weights are weight average molecular weights.

Except where specific examples of actual measured values are presented, numerical values referred to herein should be considered to be qualified by the word "about".

The present invention relates to emulsions having a concentrated internal or discontinuous oil phase, which represents at least 50% by weight of the emulsion, preferably at least 70%, more preferably at least 80% and more preferably still from 80 to 93% by weight of the emulsion.

The oil phase according to the invention may comprise any water immiscible material that is liquid at ambient conditions; any material that is solid at ambient conditions, has a melting temperature of less than 100°C and melts to form a water immiscible liquid; mixtures of such materials.

As used herein in relation to the oil phase, the term "water immiscible" includes materials having a Hildebrand Solubility Parameter of around 5-12 calories/cc (209 - 502 kJ/m²). The solubility parameter is defined as the sum of all attractive forces radiating out of a molecule. The total Van der Waals force is called the Hildebrand Solubility Parameter and can be calculated using Hildebrand's equation using boiling point and MW data. Methods and a computer program for calculating the Hildebrand Solubility Parameter are disclosed by C.D. Vaughan in J. Cosmet. Chem. 36, 319-333 (September/October 1985).

Materials comprised within the oil phase may have any polarity and may include aliphatic or aromatic hydrocarbons, esters, alcohols, ethers, carbonates, fluorocarbons, silicones, fluorosilicones or derivatives thereof.

Solid materials that may be present in the oil phase include waxes. As used herein, the term "wax" includes natural and synthetic waxes. The class of natural waxes includes animal waxes, such as beeswax, lanolin, shellac wax and Chinese insect wax; vegetable waxes, such as carnauba, candelilla, bayberry and sugar cane; mineral waxes, such as ceresin and ozokerite; petrochemical waxes, such as microcrystalline wax and petrolatum. The class of synthetic waxes includes ethylenic polymers and polyol ether-esters, chlorinated naphthalenes and Fischer-Tropsch waxes. For more details, please refer to see Römpp Chemie Lexikon, Georg Thieme Verlag, Stuttgart, 9th Edition, 1995 under "Wachse".

Advantageously, materials comprised within the oil phase, including the melted waxes, have a viscosity in the range from 0.005 to 30,000cm²/s (0.5 to 3,000,000 cst), preferably from 0.005 to 20,000cm²/s (0.5 to 2,000,000 cst), more preferably from 0.005 to 3500cm²/s (0.5 to 350,000 cst).

The aqueous phase of the emulsions according to the invention comprises water and may also comprise additional water-soluble components, such as alcohols; humectants, including polyhydric alcohols (e.g. glycerine and propylene glycol); active agents such as d-panthenol, vitamin B₃ and its derivatives (such as niacinamide) and botanical extracts; thickeners and preservatives.

Advantageously, the emulsions according to the invention comprise substantially no electrolyte. As used herein, the term "electrolyte" includes substances that form ions in aqueous solution and the term "substantially no electrolyte" means that an emulsion according to the invention comprises less than 0.001% electrolyte by weight of the emulsion. It is beneficial to exclude electrolytes because the diluted emulsions may become much more difficult to thicken when they are present - many thickeners are highly sensitive to salt levels. Thickeners are frequently used in emulsions to increase the viscosity of the water phase, thereby reducing the ability of oil phase droplets, which typically have a lower density than the aqueous phase, to rise to the top of an emulsion formulation (so-called "creaming").

Preferably, the viscosity of the aqueous phase does not exceed 2 kg/ms (2000 cps), measured using a Brookfield Digital Rheometer Model DV-III, with an RV2 spindle at 20rpm (Brookfield Engineering Laboratories- Stoughton Ma.) at 20°C. Above this point, emulsification may become extremely difficult, especially when the internal oil phase is present at high levels, such as 80-90% by weight of the emulsion.

The water-soluble emulsification polymers according to the invention have a molecular weight of at least 500 Daltons, since below this level, the resulting emulsions have poor skin feel. Skin feel improves with increasing molecular weight and it is preferred that the water-soluble emulsification polymers according to the invention have a molecular weight above 3000 Daltons, more preferably above 9000 Daltons and more preferably still, above 10,000 Daltons.

At the levels of emulsifier present in the emulsions according to the invention, the molecular weight of the emulsification polymers advantageously does not exceed 130 kiloDaltons; above this point, the viscosity of the aqueous phase may reach a level that hinders emulsification, especially when the internal oil phase is present at levels of 80-90% by weight of the emulsion.

Advantageously, from 70% to 100% of the total weight of emulsifier comprised within the present emulsions consists of one or more non-alkoxylated water-soluble emulsification polymers.

Surprisingly, it has been found that any non-alkoxylated, water-soluble polymer fulfilling the defined molecular weight and surface tension criteria may be used to emulsify the emulsions according to the present invention and are capable of mitigating the problems encountered in the prior art. This applies regardless of the chemical nature of the water-soluble polymer, so that polymers of widely differing chemistries may be employed. Non-limiting water-soluble polymers which may be employed according to the invention include: alkylated polyvinylpyrrolidone, such as buylated polyvinylpyrrolidone commercialised as "Ganex P904" by ISP Corp.; mono alkyl esters of poly(methyl vinyl ether/maleic acid) sodium salt, including mono butyl ester of poly(methyl vinyl maleic acid sodium salt) such as included in the product commercialised as "EZ Sperse" by ISP Corp; isobutylene/ethylmaleimide/hydroxyethyl copolymer, such as included in the product commercialised as "Aquafix FX64" by ISP Corp.; (3-dimethylaminopropyl)-methacrylamide/3-methacryloylamidopropyl-lauryl-dimthyl-ammonium chloride, such as included in the product commercialised as Styleze W20 by ISP Corp, and mixtures thereof.

Advantageously, at least one of the non-alkoxylated, water-soluble polymers according to the invention has film-forming properties. These properties are found in higher molecular weight polymers, especially those having a molecular weight above 10,000 Daltons. The film-forming property may further increase the substantivity of the emulsions on the substrate versus traditional surfactants, including alkoxylated surfactants. Dried-down oil-in-water emulsions comprising traditional surfactants, including alkoxylated surfactants, suffer from the disadvantage that they may re-emulsify when wetted, whereas the present non-alkoxylated, water-soluble polymers are less liable to do that. Without wishing to be bound by theory, it is believed that the substantivity of the present compositions may be further increased if the polymers exhibit film-forming properties, because the film-forming polymer may form a film over the oil phase to retain it on the substrate.

The emulsions according to the invention may comprise from 0.1% to 15%, preferably from 0.1% to 5% and more preferably 0.1 to 2.5% by weight water-soluble emulsification polymer.

The emulsions according to the invention may be manufactured in the following way:

A typical emulsion might contain 1-5% water-soluble emulsification polymer and 8-9% aqueous phase, the aqueous phase comprising 100% water or a mixture of water and other water-soluble components. In a first step, the water-soluble emulsification polymer is added to the aqueous phase with mixing. Following this, discrete batches of 6-15% of the total weight of oil are titrated sequentially into the aqueous phase accompanied by gentle mixing to obtain a uniform consistency prior to addition of the following batch. This is continued until around 20% of the total weight of oil has been added. At this point the remainder of the oil may be added more rapidly and in a continuous fashion with more vigorous mixing until a uniform emulsion comprising all the oil is obtained. Mixing is continued until a uniform consistency is obtained exhibiting a typical particle in a desired range. A typical particle size would be in the range from I to 20 microns. The concentrated emulsion obtained typically comprises above 70%, and more often from 80 to 93% internal oil phase by weight of the emulsion.

The emulsions according to the invention may be employed as products in their own right. Make-up remover gels, for example, may comprise the present concentrated emulsion. In addition, however, the present emulsions may be incorporated into other products as components thereof. According to a second aspect of the invention, products are provided comprising the emulsions according to the first aspect of the invention. Examples of such products include personal care products, such as lotions for hand and body, shampoo compositions, make-up, perfume and perfume gel compositions and lotions for baby wipes; laundry products such as fabric softener and liquid laundry detergent; health care products, such as vapour rub creams; coatings for tissue towels.

Personal care, health care and laundry products may comprise from 0.01 to 30%wt, preferably from 0.25 to 12%wt, more preferably 0.25 to 5%wt of the above-defined concentrated emulsions.

Non-limiting examples of materials that may be included in such products are thickeners; surfactants, such as non-ionic, anionic, cationic, zwitterionic and amphoteric surfactants; humectants, such as polyhydric alcohols, including glycerine and propylene glycol; pigments, including organic and inorganic pigments; preservatives; chelating agents, antimicrobials, perfumes.

### Measurement Methods

### Testing the solubility of the water-soluble emulsification polymers

As used herein in relation to the emulsification polymers, the term "water-soluble" includes polymers fulfilling the following condition: a 1%wt solution of the polymer in de-ionised water at room temperature gives at least 90% transmittance of light having a wavelength in the range from 455 to 800nm. Testing was carried out by passing the polymer solution through a standard syringe filter into a 1cm path length cuvette having a pore size of 450 nm and scanning using an HP 8453 Spectrophotometer arranged to scan and record across 390 to 800 nm. Filtration was carried out to remove insoluble components.

### Measurement of surface tension

The method used for measuring surface tension of fluid is the so-called "Wilhelmy Plate Method". The Wilhelmy plate method is a universal method especially suited to establishing surface tension over time intervals. In essence, a vertical plate of known perimeter is attached to a balance, and the force due to wetting is measured. More specifically:

A 0.1%wt aqueous solution of water-soluble emulsification polymer is made up in de-ionised water. The polymer solution is then poured into a clean and dry glass vessel, the solution temperature being controlled at 25°C. The clean and annealed Wilhelmy Plate is lowered to the surface of the liquid. Once the plate has reached the surface the force which is needed to remove the plate out of the liquid is measured.

The equipment used and corresponding settings are as follows:
Device: Krüss Tensiometer K12, manufactured by Krüss GmbH, Borsteler Chausee 85-99a , 22453 Hamburg- Germany (see www.kruess.com).
Plate Dimensions: Width: 19.9mm; Thickness: 0.2mm; Height: 10mm
Measurement Settings: immersion depth 2mm, Surface Detection Sensitivity 0.01g, Surface Detection Speed 6mm/min, Values 10, Acquisition linear, Maximum Measurement Time 60sec

The plate is immersed in the fluid and the corresponding value of surface tension is read on the display of the device. Instructions can be found in the user manual edited by "Krüss GmbH Hamburg 1996" Version 2.1.

### Examples

The following examples further describe and demonstrate the preferred embodiments within the scope of the present invention. The examples are given solely for the purpose of illustration, and are not to be construed as limitations of the present invention since many variations thereof are possible without departing from its scope.

### Example 1: Hand and Body Lotion

| **Concentrated Oil-In-Water Emulsion** | | |
|---|---|---|
| **Material** | **%wt** | **Weight (g)** |
| Isohexadecane | 58.37 | 583.7 |
| Isopropyl isostearate | 14.60 | 146.0 |
| DL tocopheryl acetate | 2.43 | 243 |
| Dow Coming 1503¹ | 14.60 | 146.0 |
| EZ Sperse² | 4.00 | 40.0 |
| Water | 6.00 | 60.0 |

| | | |
|---|---|---|
| ¹Dow Coming 1503 fluid is a dimethicone and dimethiconol produced by Dow Coming ²EZ Sperse is a 25% solution of mono butyl ester of poly(methyl vinyl maleic acid sodium salt) and is a copolymer of maleic anhydride and methyl vinyl ether reacted with water/butanol to form a half ester, which is neutralised with sodium hydroxide. EZ Sperse is produced by ISP Corp. | | |

### Procedure to make a 1000g batch of concentrated oil-in-water emulsion

Isohexadecane, isopropyl isostearate and DL-tocopheryl acetate were mixed using a Kitchen Aid Ultra Power Mixer until uniform. The water and EZ Sperse were mixed in the same way. The isohexadecane/isopropyl isostearate/DL-tocopheryl acetate were then added to the water/EZ Sperse at a rate of 8g/minute while continually mixing with a Kitchen Aid Ultra Power Mixer having a paddle attachment at a setting of "4". Following complete addition, the Dow Coming 1503 was added to the mixture at the same rate and mixed in the same way until a uniform mixture was obtained.

| **Hand and Body Lotion** | | |
|---|---|---|
| **Material** | **%wt** | **Weight (g)** |
| Deionised water | 69.92 | 699.2 |
| Glycerine | 5.00 | 50.0 |
| Phenonip³ | 1.00 | 10.0 |
| D-Panthenol | 0.50 | 5.0 |
| Sepigel 305⁴ | 4.00 | 40.0 |
| System 3 AM900⁵ | 5.33 | 53.3 |
| System 3 AM500⁶ | 4.00 | 40.0 |
| Concentrated oil-in-water emulsion | 10.28 | 102.8 |

| | | |
|---|---|---|
| ³Phenoxyethanol and Methyl-, Ethyl-, Buyl-, Popyl and Isobutylparaben from Nipa Labs Inc. ⁴Seppigel 305 is polyacrylamide & C13-14 isoparaffin & laureth-7 and is available from Seppic Group. ⁵System 3 AM500 is a mixture of water, petrolatum, lecithin, hydrogenated lecithin, and polyphosphorylcholine glycol acrylate commercialised by Collaborative Laboratories Inc. ⁶System 3 AM900 is a mixture of water, cetearyl alcohol, hydrogenated polyisobutene, lecithin, hydrogenated lecithin, butylene glycol and polyphosphorylcholine glycol acrylate commercialised by Collaborative Laboratories Inc. | | |

### Procedure to make a 1000g batch of hand and body lotion

All mixing was carried out using a Kitchen Aid Ultra Power Mixer with a paddle attachment and a speed setting of "2".

The de-ionised water, glycerine, Phenonip and d-panthenol were mixed until uniform at which point the Sepigel 305 was dispersed into the mixture and also mixed until uniform. Following this, the System 3 AM900 was added to the mixture and mixed until uniform, the System 3 AM500 was added and mixed until uniform and finally the concentrated oil-in-water emulsion was added to the mixture and mixed in to create a hand and body lotion of uniform consistency.

### Example 2: Make-up foundation

| **Pigment Pre-Mix** | |
|---|---|
| **Material** | **%wt** |
| Water | 2.000 |
| Ganex P904⁵ | 8.000 |
| BTD 401¹ | 9.075 |
| BEYO 12² | 0.811 |
| BERO 12³ | 0.262 |
| BEBO 12⁴ | 0.143 |

| | |
|---|---|
| ¹Kobo Products Inc., titanium dioxide and isopropyl titanium triisostearate ²Kobo Products Inc., hydrated ferric oxide and isopropyl titanium triisostearate ³Kobo Products Inc., ferric oxide and isopropyl titanium triisostearate ⁴Kobo Products Inc., iron oxide and isopropyl titanium triisostearate ⁵Butylated polyvinylpyrollidone obtained from ISP Corp., Inc. | |

### Procedure to make pigment pre-mix

The Ganex P904 and water were mixed using a Kitchen Aid Ultra Power Mixer until uniform. The pigments were then added to the Ganex P904/water and mixed using a Cito Unguator mixer for 1 minute at a setting of 5.

| **Concentrated Oil-In-Water Emulsion** | | |
|---|---|---|
| **Material** | **%wt** | **Weight (g)** |
| Tridecyl neopentonate | 23.69 | 236.9 |
| Dow Coming 246 Fluid⁶ | 56.83 | 568.3 |
| Dow Corning 245 Fluid⁷ | 9.48 | 94.8 |
| EZ Sperse | 2.50 | 25.0 |
| Water | 7.50 | 75.0 |

| | | |
|---|---|---|
| ⁶Cyclohexsiloxane fluid produced by Dow Coming ⁷Cyclopentasiloxane fluid produced by Dow Coming | | |

### Procedure to make a 1000g batch of concentrated oil-in-water emulsion

Tridecyl neopentanoate, Dow Coming 245 and 246 were mixed using a Kitchen Aid Ultra Power Mixer until uniform. The water and EZ Sperse were mixed in the same way. The Tridecyl neopentanoate/Dow Coming 245 and 246 were then added to water/EZ Sperse at a rate of 8g/minute while continually mixing with a Kitchen Aid Ultra Power Mixer having a paddle attachment at a setting of "4".

| **Make-Up Foundation** | |
|---|---|
| **Material** | **%wt** |
| Water | 45.7 |
| Phenonip | 1.0 |
| Glycerine | 6.0 |
| Dry Flow Elite BN⁸ | 3.0 |
| Seppigel 305 | 3.0 |
| Pigment Pre-Mix | 20.3 |
| Concentrated oil-in-water emulsion | 21.0 |

| | |
|---|---|
| ⁸Aluminium starch octylsuccinate and boron nitride obtainable from National Starch & Chemical | |

### Procedure to make make-up foundation

All mixing was carried out using a Kitchen Aid Ultra Power Mixer with a paddle attachment and a speed setting of "2".

The Phenonip was dispersed in the water and mixed. The Dry Flo Elite BN was dispersed in the glycerine and mixed until uniform, then the glycerine/Dry Flow Elite BN was added to the Phenonip/water and mixed until uniform. The Seppigel 305 was added to the mixture and mixed until smooth and lump free. At this point, the pigment was mixed in and mixing was continued until the colour was uniform. Lastly, the concentrated oil-in-water emulsion was mixed in until uniform to create the finished make-up foundation.

### Example 3: perfume gel

| **Concentrated Oil-In-Water Emulsion** | | |
|---|---|---|
| **Material** | **%wt** | **Weight (g)** |
| EZ Sperse | 2.5 | 25 |
| Water | 17.5 | 175 |
| Fragrance Oil | 80.0 | 800 |

### Procedure to make the concentrated oil-in-water emulsion

The EZ Sperse and 7.5%wt water were mixed using a Kitchen Aid Ultra Power Mixer until uniform. The fragrance oil was then added at a rate of 8g/minute while continually mixing with a Kitchen Aid Ultra Power Mixer having a paddle attachment at a setting of "4". Due to the high viscosity, the emulsion was then diluted with the remainder of the water while continually mixing with a Kitchen Aid Ultra Power Mixer having a paddle attachment at a setting of "4".

| **Perfume Gel** | |
|---|---|
| **Material** | **%wt** |
| Deionised water | 86.5 |
| Seppigel 305 | 3.5 |
| Concentrated perfume o/w emulsion | 10.0 |

### Procedure to make perfume gel

All mixing was carried out using a Kitchen Aid Ultra Power Mixer with a paddle attachment and a speed setting of "2".

The Seppigel 305 was dispersed in the water and mixed until a smooth gel was obtained, at which point the perfume o/w emulsion was added and mixed until a uniform consistency perfume gel was produced.

### Example 4: make-up remover gel

| **Concentrated Oil-In-Water Emulsion and Transfer-Resistant Make-Up Remover Gel** | | |
|---|---|---|
| **Material** | **%wt** | **Weight (g)** |
| Ganex P904 | 1 | 10 |
| De-ionised water | 9 | 90 |
| Isohexadecane | 85 | 850 |
| Glycerine (99%) | 5 | 50 |

All mixing was carried out using a Kitchen Aid Ultra Power Mixer with a paddle attachment and a speed setting of "2".

The Ganex P904 was dissolved in the de-ionised water with mixing. Isohexadecane was added into the Ganex P904/water at a rate of 8g/minute and mixed until uniform. Lastly, the glycerine was added to the mixture and the concentrated emulsion was again mixed to create a transfer-resistant make-up remover gel.

### Example 5: Baby Vapour Rub Cream

| **Essential Oil Mix** | |
|---|---|
| **Material** | **%wt** |
| Eucalyptus oil | 76.79 |
| Rosemary oil | 13.39 |
| Aloe vera oil extract | 8.93 |
| Lavender Oil | 0.89 |

The essential oils were mixed uniformly, then incorporated into an oil-in-water emulsion having the following composition:

| **Essential Oil Concentrated Oil-In-Water Emulsion** | |
|---|---|
| **Material** | **%wt** |
| EZSperse | 10 |
| Water | 10 |
| Essential Oil Mix | 80 |

Water and EZ Sperse were mixed using a Kitchen Aid Ultra Power Mixer until uniform. The essential oils were then added to the water/EZ Sperse while continually mixing with a Kitchen Aid Ultra Power Mixer having a paddle attachment at a setting of "4" until a uniform oil-in-water emulsion was obtained.

| **Baby Vapour Rub Cream** | |
|---|---|
| **Material** | **%wt** |
| Deionised water | 69.80 |
| Glycerine | 5.00 |
| 1,2-hexanediol | 2.00 |
| Ethyl paraben | 0.14 |
| Benzyl alcohol | 0.25 |
| Luvigel EM (BASF)¹ | 4.90 |
| Brij 30² | 0.10 |
| AM 900 | 4.50 |
| Essential oil concentrated oil-in-water emulsion | 13.30 |

| | |
|---|---|
| ¹Caprylic/Capric Triglyceride (and) Sodium Acrylates Copolymer from BASF ²Laureth-4 from Uniquema | |

All mixing was carried out using a Kitchen Aid Ultra Power Mixer.

The following premixes were prepared:
A. Glycerine and water
B. 1,2-hexanediol, ethyl paraben and benzyl alcohol
C. Luvigel EM and Brij 30

Following preparation of the premixes, premix A was added to premix B with mixing until uniform. After this, premix C was added and mixed until a smooth creamy consistency had been achieved, at which point the AM 900 was incorporated, again with mixing. Finally, the concentrated oil-in-water emulsion was added and the whole was mixed to achieve a uniform consistency baby vapour rub cream.

### Example 6: Topical Analgesic Composition

| **Analgesic Active Pre-Mix** | |
|---|---|
| **Material** | **%wt** |
| Methyl salicylate | 75 |
| Menthol | 25 |

The pre-mix components are thoroughly mixed using a Kitchen Aid Ultra Power Mixer until all the menthol has dissolved.

| **Concentrated Oil-In-Water Emulsion** | |
|---|---|
| **Material** | **%wt** |
| EZ Sperse | 10 |
| Water | 10 |
| Analgesic Active Pre-Mix | 80 |

Water and EZ Sperse were mixed using a Kitchen Aid Ultra Power Mixer until uniform. The analgesic active pre-mix was then added to the water/EZ Sperse while continually mixing with a Kitchen Aid Ultra Power Mixer having a paddle attachment at a setting of "4" until a uniform oil-in-water emulsion was obtained.

| **Topical Analgesic Active Cream** | |
|---|---|
| **Material** | **%wt** |
| Deionised water | 64.36 |
| Glycerine | 5.00 |
| 1,2-hexanediol | 2.00 |
| Ethyl paraben | 0.14 |
| Benzyl alcohol | 0.25 |
| Luvigel EM (BASF)¹ | 4.90 |
| Brij 30² | 0.10 |
| AM 900 | 4.50 |
| Concentrated oil-in-water emulsion | 18.75 |

All mixing was carried out using a Kitchen Aid Ultra Power Mixer.

The following premixes were prepared:
A. Glycerine and water
B. 1,2-hexanediol, ethyl paraben and benzyl alcohol
C. Luvigel EM and Brij 30

Following preparation of the premixes, premix A was added to premix B with mixing until uniform. After this, premix C was added and mixed until a smooth creamy consistency had been achieved, at which point the AM 900 was incorporated, again with mixing. Finally, the concentrated oil-in-water emulsion was added and the whole was mixed to achieve a uniform consistency topical analgesic active cream.

### Example 7: Anti-Acne Gel

| **Anti-Acne Active Pre-Mix** | |
|---|---|
| **Material** | **%wt** |
| Arlamol E¹ | 80 |
| Salicylic Acid | 20 |

| | |
|---|---|
| ¹PPG-15 Stearyl Ether from Uniquema. | |

The pre-mix components were heated to 50°C and thoroughly mixed using a propeller mixer until the salicylic acid had dissolved to leave a clear solution.

| **Concentrated Oil-In-Water Emulsion** | |
|---|---|
| **Material** | **%wt** |
| Water | 19.1 |
| Ganex P904 | 0.9 |
| Anti-Acne Pre-Mix | 80 |

Water and Ganex P904 were mixed using a Kitchen Aid Ultra Power Mixer until uniform. The anti-acne active pre-mix was then added to the water/Ganex P904 while continually mixing with a Kitchen Aid Ultra Power Mixer having a paddle attachment at a setting of "4" until a uniform oil-in-water emulsion was obtained.

| **Anti-Acne Gel** | |
|---|---|
| **Material** | **%wt** |
| Deionised water | 71 |
| Seppigel 305 | 4 |
| Concentrated oil-in-water emulsion | 25 |

All mixing was carried out using a Kitchen Aid Ultra Power Mixer.

The Seppigel 305 was added to the water and mixed until a viscous uniform dispersion was obtained. The concentrated oil-in-water emulsion was then added to the Seppigel 305/water and mixed until a uniform consistency anti-acne gel was obtained.

### Example 8: wax emulsion

| **Concentrated Oil-In-Water Emulsion** | | |
|---|---|---|
| **Material** | **%wt** | **Weight (g)** |
| EZSperse | 5.0 | 50 |
| Water | 5.0 | 50 |
| Glycerine (99%) | 10.0 | 100 |
| USP Petrolatum | 80.0 | 800 |

### Procedure to make oil-in-water emulsion

The EZSperse, water and glycerine are mixed until uniform. This mixture was heated to 70°C. Separately, the petrolatum was also heated to 70°C. The petrolatum was then slowly added to the aqueous phase and continuously mixed with a Kitchen Aid Mixer equipped with a paddle blade. Mixing was continued until a uniform consistency was obtained.

## Claims

1. Emulsion comprising at least 50% by weight of the emulsion of discontinuous oil phase, an aqueous continuous phase and emulsifier, wherein the emulsifier comprises a non-alkoxylated water-soluble emulsification polymer having a molecular weight of at least 500 Daltons, a 0.1 %wt aqueous solution of the water-soluble emulsification polymer having a surface tension of 15-60 mN/m (15-60 dynes/cm) when measured at 25°C, wherein the non-alkoxylated water-soluble emulsification polymer comprises no -OR groups, where R includes alkyl moieties, in the molecule, either in the polymer backbone, as pendants thereto, or elsewhere.

2. Emulsion according to claim 1, wherein from 70% to 100% of the total weight of emulsifier consists of one or more non-alkoxylated water-soluble emulsification polymers.

3. Emulsion according to claim 1 or 2, comprising greater than 70%wt oil phase, preferably at least 80%wt oil phase, more preferably from 80-93%wt oil phase.

4. Emulsion according to any one of the preceding claims, wherein the or each oil comprised within the oil phase has a Hildebrand Solubility Parameter of around 5-12 calories/cc (209 - 502 kJoule/m²).

5. Emulsion according to any one of the preceding claims, wherein the or each oil comprised within the oil phase has a viscosity in the range from 0.005cm²/s to 30,000cm²/s.

6. Emulsion according to any one of the preceding claims, wherein the or each water-soluble emulsification polymer has a molecular weight above 3000 Daltons, preferably above 10,000 Daltons.

7. Emulsion according to any one of the preceding claims, wherein the or each emulsification polymer has a molecular weight of less than 130 kiloDaltons.

8. Emulsion according to any one of the preceding claims, comprising from 0.25 to 7%wt, preferably from 0.25 to 5%wt of emulsification polymer.

9. Emulsion according to any one of the preceding claims, wherein at least one of the one or more water-soluble emulsification polymers is a film-forming polymer.

10. Emulsion according to any one of the preceding claims, wherein the water-soluble emulsification polymer comprises mono alkyl esters of poly(methyl vinyl ether/maleic acid) sodium salt, alkylated polyvinylpyrrolidone, (3-dimethylaminopropyl)-methacrylamide/3-methacryloylamidopropyl-lauryl-dimethyl-ammonium chloride, or mixtures of these materials.

11. Emulsion according to any one of the preceding claims which comprises less than 0.001 % by weight of the emulsion of electrolyte.

12. Personal care, health care or laundry product comprising from 0.01 to 30%wt, preferably from 0.25 to 5%wt of the emulsion according to any one of the preceding claims.

13. Personal care product according to claim 12, wherein the product is a lotion, a shampoo, a make-up or a perfume gel.

14. Lotion according to claim 13, wherein the lotion is a hand and body lotion.

15. Health care product according to claim 12, wherein the product is a vapour rub cream.

## Patentansprüche

1. Emulsion, umfassend zu mindestens 50 Gew.-% der Emulsion eine diskontinuierliche Ölphase, eine wässrige kontinuierliche Phase und einen Emulgator, wobei der Emulgator ein nichtalkoxyliertes wasserlösliches emulgierendes Polymer mit einem Molekulargewicht von mindestens 500 Dalton umfasst, wobei eine 0,1-gew.-%-ige wässrige Lösung des wasserlöslichen emulgierenden Polymers eine Oberflächenspannung von 15-60 mN/m (15-60 Dyn/cm) hat, wenn bei ungefähr 25 °C gemessen wird, wobei das nichtalkoxylierte wasserlösliche emulgierende Polymer keine -OR-Gruppen umfasst, wobei R Alkyleinheiten in dem Molekül, entweder in der Polymerhauptkette, seitenständig daran oder anderswo einschließt.

2. Emulsion nach Anspruch 1, wobei von 70 Gew.-% bis 100 Gew.-% des Gesamtgewichts an Emulgator aus einem oder mehreren nichtalkoxylierten wasserlöslichen emulgierenden Polymeren bestehen.

3. Emulsion nach Anspruch 1 oder 2, umfassend zu mehr als 70 Gew.-% eine Ölphase, vorzugsweise umfassend zu mindestens 80 Gew.-% eine Ölphase, mehr bevorzugt von 80-93 Gew.-% eine Ölphase.

4. Emulsion nach einem der vorstehenden Ansprüche, wobei das oder jedes Öl, das innerhalb der Ölphase enthalten ist, einen Hildebrandschen Löslichkeitsparameter von ungefähr 5-12 Kalorien/cm³ (209-502 kJoule/m²) aufweist.

5. Emulsion nach einem der vorstehenden Ansprüche, wobei das oder jedes Öl, das innerhalb der Ölphase enthalten ist, eine Viskosität im Bereich von 0,005 cm²/s bis 30.000 cm²/s aufweist.

6. Emulsion nach einem der vorstehenden Ansprüche, wobei das wasserlösliche emulgierende Polymer ein Molekulargewicht über 3000 Dalton, vorzugsweise über 10.000 Dalton aufweist.

7. Emulsion nach einem der vorstehenden Ansprüche, wobei das oder jedes emulgierende Polymer ein Molekulargewicht von weniger als 130 Kilodalton aufweist.

8. Emulsion nach einem der vorstehenden Ansprüche, umfassend von 0,25 bis 7 Gew.-%, vorzugsweise von 0,25 bis 5 Gew.-% das emulgierende Polymer.

9. Emulsion nach einem der vorstehenden Ansprüche, wobei mindestens eines des einen oder der mehreren wasserlöslichen emulgierenden Polymere ein filmbildendes Polymer ist.

10. Emulsion nach einem der vorstehenden Ansprüche, wobei das wasserlösliche emulgierende Polymer Monoalkylester von Poly(methylvinylether/maleinsäure)-Natriumsalz, alkyliertes Polyvinylpyrrolidon, (3-Dimethylaminopropyl)-methacrylamid/3-Methacryloylamidopropyl-lauryl-dimethylammoniumchlorid oder Mischungen dieser Materialien umfasst.

11. Emulsion nach einem der vorstehenden Ansprüche, die zu weniger als 0,001 Gew.-% der Emulsion Elektrolyt umfasst.

12. Körperpflege-, Gesundheitspflege- oder Wäschewaschprodukt, umfassend von 0,01 bis 30 Gew.-%, vorzugsweise von 0,25 bis 5 Gew.-% die Emulsion nach einem der vorstehenden Ansprüche.

13. Körperpflegeprodukt nach Anspruch 12, wobei das Produkt eine Lotion, ein Shampoo, ein Make-up oder ein Parfümgel ist.

14. Lotion nach Anspruch 13, wobei die Lotion eine Hand- und Körperlotion ist.

15. Gesundheitspflegeprodukt nach Anspruch 12, wobei das Produkt eine Inhalationssalbe ist.

## Revendications

1. Émulsion comprenant au moins 50 % en poids de l'émulsion d'une phase huileuse discontinue, une phase continue aqueuse et un émulsifiant, dans laquelle l'émulsifiant comprend un polymère d'émulsification hydrosoluble non-alcoxylé ayant une masse moléculaire d'au moins 500 Daltons, une solution aqueuse à 0,1 % en poids du polymère d'émulsification hydrosoluble ayant une tension superficielle de 15 à à 60 mN/m (15 à 60 dynes/cm) lorsqu'elle est mesurée à environ 25 °C, et dans laquelle la solution aqueuse du polymère d'émulsification hydrosoluble non-alcoxylé ne comprend aucun groupe -OR, où R inclut des fragments alkyle, dans la molécule, ou dans le squelette polymère, en tant que greffes à celui-ci, ou ailleurs.

2. Émulsion selon la revendication 1, dans laquelle de 70 % à 100 % du poids total de l'émulsifiant est constitué d'un ou plusieurs polymères d'émulsification hydrosolubles non-alcoxylés.

3. Émulsion selon la revendication 1 ou 2, comprenant plus de 70 % en poids de phase huileuse, de préférence comprenant au moins 80 % en poids de phase huileuse, plus préférablement de 80 à 93 % en poids de phase huileuse.

4. Émulsion selon l'une quelconque des revendications précédentes, dans laquelle la, ou chaque huile, comprise au sein de la phase huileuse a un paramètre de solubilité d'Hildebrand d'environ 5 à 12 calories/cm³ (209 à 502 kJoule/m²).

5. Émulsion selon l'une quelconque des revendications précédentes, dans laquelle la, ou chaque huile, comprise au sein de la phase huileuse a une viscosité dans la gamme de 0,005 cm²/s à 30 000 cm²/s.

6. Émulsion selon l'une quelconque des revendications précédentes, dans laquelle le polymère d'émulsification hydrosoluble a une masse moléculaire supérieure à 3000 Daltons, de préférence supérieure à 10 000 Daltons.

7. Émulsion selon l'une quelconque des revendications précédentes, dans laquelle le polymère d'émulsification a une masse moléculaire inférieure à 130 kiloDaltons.

8. Émulsion selon l'une quelconque des revendications précédentes, comprenant de 0,25 à 7 % en poids, de préférence de 0,25 à 5 % en poids du polymère d'émulsification.

9. Émulsion selon l'une quelconque des revendications précédentes, dans laquelle au moins un des uns ou plusieurs polymères d'émulsification hydrosolubles est un polymère filmogène.

10. Émulsion selon l'une quelconque des revendications précédentes, dans laquelle le polymère d'émulsification hydrosoluble est choisi dans le groupe constitué de monoalkyl esters de sel de sodium de poly(méthyl-vinyl éther/acide maléique), polyvinylpyrrolidone alkylée, chlorure de (3-diméthylaminopropyl)-méthacrylamide/3-méthacryloylamidopropyl-lauryl-diméthyl-ammonium, ou des mélanges de ces matériaux.

11. Émulsion selon l'une quelconque des revendications précédentes, qui comprend moins de 0,001 % en poids de l'émulsion d'un électrolyte.

12. Produits pour les soins d'hygiène personnelle, les soins de santé ou le lavage du linge comprenant de 0,01 à 30 % en poids, de préférence de 0,25 à 5 % en poids de l'émulsion selon l'une quelconque des revendications précédentes.

13. Produit de soins d'hygiène personnelle selon la revendication 12, où le produit est une lotion, un shampooing, un maquillage et un gel parfumé.

14. Lotion selon la revendication 13, où la lotion est une lotion pour les mains et le corps.

15. Produit pour les soins de santé selon la revendication 12, où le produit est une crème décongestionnante.
